# EUROPEAN PATENT APPLICATION

(11) **EP 1 413 202 A1**
(43) Date of publication of application: **28.04.2004**
(21) Application number: 02079422.8
(22) Date of filing: 22.10.2002
(51) Int. Cl.: A21D 8/04, A21D 2/02, A21D 2/18, C12N 9/98, A23P 1/04, A23L 1/22, A23P 1/08

(54) **Lipid-encapsulated functional bakery ingredients**

(71) Applicant: CSM Nederland B.V., 1112 XE Diemen (NL)
(72) Inventor: Düsterhoft, Eva-Maria, NL-6718 ZB Ede (NL); Minor Marcel, NL-6704 AA Wageningen (NL); Nikolai, Karin, A-9210 Portschach (AT); Hargreaves, Neil Graham, Chester, CH4 8AE (GB); Huscroft, Simon Christopher, Brooklands, Sale M33 3TR (GB); Scharf, Udo, D-55413 Weiler (DE)
(74) Representative: van Westenbrugge, Andries

(57) **Abstract**

The present invention is concerned with lipid-encapsulated or lipid-coated functional bakery ingredients. More particularly, the invention relates to a granule suitable for use in the preparation of a dough, comprising:
a. a hydrophilic core with a diameter of at least 5 µm, said core containing a functional bakery ingredient selected from the group of enzymes, oxidoreductants, acidulants, hydrocolloids, starches, yeast, sugars, water, flavours and combinations thereof; and
b. a lipophilic substantially continuous layer encapsulating the core, which layer contains at least 50 wt.% triglyceride fat with a slip melting point of at least 30°C and at least 1 wt.% of a release agent selected from the group of monoglycerides, diglycerides, datems, lactems, citrems, stearyl-lactylates, polyglycerol esters, lecithins, sucrose esters, fatty acids, soaps and combination thereof.

Other aspects of the invention relate to methods for preparing the aforementioned encapsulated or coated ingredients and the use of these lipid-encapsulated or lipid-coated ingredients in the preparation of a dough composition.

## Description

### TECHNICAL FIELD OF THE INVENTION

The present invention is concerned with lipid-encapsulated or lipid-coated functional bakery ingredients, methods for preparing such encapsulated or coated ingredients and the use of these lipid-encapsulated or lipid-coated ingredients in the preparation of a dough composition.

### BACKGROUND OF THE INVENTION

Functional bakery ingredients are widely used in the baking industry to improve handling and machinability of doughs and also to improve texture, volume, flavour, and freshness (anti-staling) of the final baked product. Examples of functional bakery ingredients that can be used to "condition" a dough include enzymes, oxidoreductants, acidulants, hydrocolloids, starches, yeast, sugars, water and flavours.

An important area of application of functional bakery ingredients is bread. Bread is made from four principal ingredients: flour, yeast, salt and water. It is usually prepared in three basic steps, and the end result is a baked loaf. The steps are: (a) the principal ingredients are mixed to form a dough and worked to develop a continuous visco-elastic gluten matrix; (b) the developed dough is then proved by incubation in warm, humid conditions to promote fermentation by the yeast causing the dough to rise; (c) the risen dough is then baked to gelatinise starch, denature protein and fix the dough structure. Various additives, including the aforementioned functional bakery ingredients, are known to improve dough development and the quality of the baked loaf. These additives are generally known as bread (or flour or dough) improvers/conditioners.

The strength of a dough is an important aspect of baking for both small-scale and large-scale applications. A strong dough has a greater tolerance of mixing time, proving time, and mechanical vibrations during dough transport, whereas a weak dough is less tolerant to these treatments. A strong dough with superior rheological and handling properties results from flour containing a strong gluten network. Flour with a low protein content or a poor gluten quality results in a weak dough.

Non-specific oxidants, such as iodates, peroxides, ascorbic acid, potassium bromate, glutathione and azodicarbonamide have a gluten strengthening effect. It has been suggested that these dough improvers induce the formation of interprotein bonds which strengthen the gluten and thereby the dough. The use of several of the currently available chemical oxidising agents has been met with consumer resistance or is not permitted by regulatory agencies.

The use of enzymes as dough improvers has been considered as an alternative to the chemical conditioners. A number of enzymes have been used recently as dough and/or bread improving agents, in particular enzymes that act on components present in large amounts in the dough. Examples of such enzymes are found within the groups of amylases, xylanases, proteases, glucose oxidases, oxygenases, redoxidases, trans-glutaminases and (hemi) cellulases, including pentosanases.

The use of the aforementioned dough improvers is not uncomplicated, since these functional ingredients tend to affect dough properties such as stickiness, strength, or stability. As a result, the dough can become difficult to handle both by hand and by machines. It would thus be desirable to be able to delay the moment when the conditioner exerts its functionality until after a selected point in time. In particular, it would be desirable to delay such a moment until all dough ingredients have been mixed and especially until such time that proving of said dough has commenced.

The lipid-encapsulation or lipid-coating of food ingredients is known in the art. US 3,561,975 describes a pie crust shrinkage-reduction agent which maintains good handling properties before baking, said agent consisting of substantially spherical particles each comprising shortening having embedded regularly therein proteolytic enzyme particles, said spherical particles having diameters ranging from about 150 microns to about 1.5 millimeters, said shortening comprising triglyceride having a complete melting point from about 95° to about 155°F (35.0-68.3°C), the weight ratio of said shortening to said enzyme ranging from about 20 to 1 to about 1 to 1. The US-patent furthermore discloses the incorporation of sorbitan fatty glyceride polyoxyethylene derivatives (Tween ®) in an amount of 11% by weight of the triglyceride. The enzyme particles within the spherical particles are said to have a longest dimension ranging from about 5 to about 150 microns, preferably ranging from about 10 to about 50 microns.

DE-A 2 203 429 is concerned with a process for the preparation of an acid composition that displays delayed dissolving behaviour, wherein a solid acid or an acid contained in a solid carrier is coated with an edible fat that is solid at ambient temperature and that contains an emulsifier. The melting point of the fat is in the range of 45°-60°C. It is stated that the emulsifier may be soy lecithin, 0.1-10% glycerol monostearate or 1-20% glycerol polyricinoleate. The acid compositions described in the German patent application are particularly useful for application in yoghurt.

US 3,716,381 describes a method of preserving meat and fish products subjected to heat treatment in a final finishing which comprises adding to the raw meat or fish a granular preservative comprising sorbic acid powder particles whose surface has been coated with a hardened oil having a melting temperature of 40°-90°C. It is observed in the US-patent that a small amount of a surfactant for food use, such as glycerol monostearate or acetylated monoglyceride, can be used along with the hardened oil.

It is an object of the present invention to provide improved lipid-encapsulated or lipid-coated functional bakery ingredient(s) that are stable under ambient conditions, wherein the encapsulation or coating controls the release of the functional bakery ingredient(s) into the dough, especially during proving of the dough.

### SUMMARY OF THE INVENTION

The inventors have discovered that the aforementioned obj ective is met by granules that comprise (a) a hydrophilic core with a diameter of at least 5 µm, which core contains the functional bakery ingredient, and (b) a lipophilic substantially continuous layer encapsulating the core, which layer contains at least 50 wt.% triglyceride fat with a slip melting point of at least 30°C and at least 1 wt.% of a release agent selected from the group of monoglycerides, diglycerides, datems, lactems, citrems, stearyl-lactylates, polyglycerol esters, lecithins, sucrose esters, fatty acids, soaps and combination thereof.

Although the inventors do not wish to be bound by theory, it is believed that the aforementioned release agents enable the swift release of the functional bakery ingredient(s) after the granules have been incorporated in the dough and the dough has reached a temperature at which a substantial part of the triglyceride fat has melted. During proving the dough usually reaches a temperature of 30-40°C and during the subsequent baking process the temperature may rise further to temperatures of up to 100°C. At these elevated temperatures, the triglyceride fat in the lipophilic encapsulating layer will melt, allowing the release agent to facilitate the access of water to the encapsulated functional bakery ingredient. Only after the subsequent release of the functional ingredient into the dough, will the functional ingredient start to exert its activity.

### DETAILED DESCRIPTION OF THE INVENTION

Accordingly, one aspect of the present invention is concerned with a granule suitable for use in the preparation of a dough, comprising:
c. a hydrophilic core with a diameter of at least 5 µm, said core containing a functional bakery ingredient selected from the group of enzymes, oxidoreductants, acidulants, hydrocolloids, starches, yeast, sugars, water, flavours and combinations thereof; and
d. a lipophilic substantially continuous layer encapsulating the core, which layer contains at least 50 wt.% triglyceride fat with a slip melting point of at least 30°C and at least 1 wt.% of a release agent selected from the group of monoglycerides, diglycerides, datems, lactems, citrems, stearyl-lactylates, polyglycerol esters, lecithins, sucrose esters, fatty acids, soaps and combination thereof.

The term "slip melting point" is defined as the temperature at which the amount of solid phase in the melting fat has become so low that an air bubble is forced upwards in an open capillary filled with the fat.

The positive impact of the above mentioned release agents is believed to be associated with their surface activity and in particular their ability to enhance the formation of a large oil-water interface once a significant part of the triglyceride fat has melted. An often-cited way to classify surfactants is by their hydrophilic/lipophilic balance, or HLB. Ranging from zero to 20, this scale indicates an surfactant's relative overall attraction to either oil or water. A low HLB indicates a strongly lipophilic emulsifier, while a high HLB indicates one that is strongly hydrophilic. In accordance with the present invention, preferably a relatively lipophilic surfactant is employed, especially a surfactant with an HLB in the range of 0-10, more preferably in the range of 1-8. In particular monoglycerides, diglycerides and stearyl lactates may advantageously be incorporated in the lipophilic layer of the granules of the present invention.

The release agent employed in accordance with the present invention preferably contains one or more fatty acid residues with, on average, 4-24 carbon atoms. Such release agents will usually display a slip melting point between 5 and 80 °C. More preferably the slip melting point of such a release agent is within the range of 20 and 70°C. Most preferably the slip melting point exceeds 65°C.

The lipophilic substantially continuous layer preferably contains from 50-98 wt.% of triglyceride fat and from 2-50 wt.% of the release agent. More preferably the lipophilic layer contains from 60-94 wt.% triglyceride fat, most preferably from 70-92 wt.%. The amount of release agent within the lipophilic layer preferably is within the range of 6-40 wt.%, more preferably of 8-30 wt.%.

In a particularly preferred embodiment of the invention the functional bakery ingredient that is contained into the core of the present granules is selected from the group consisting of enzymes, oxidoreductants and hydrocolloids. Examples of hydrocolloids include xanthan gum, guar gum, locust been gum, carrageenan, alginate, pectin, CMC, HPMC, starches and combinations thereof. Oxidoreductants that may suitably be incorporated in the core of the granules include ascorbic acid, glutathion and bromate. Typical bakery enzymes that are advantageously incorporated include α-amylase, β-amylase, xylanase, hemi-cellulase, cellulase, lipase, protease, glucose oxidase, hexose oxidase, redoxidase, lipoxygenase, peroxidase, ferrulic acid esterase, pullulanase, invertase, mananase, galactomananase, lactase and combinations thereof. Preferably the bakery enzyme is selected from the group consisting of α-amylase, xylanase and combinations thereof. Most preferably, the bakery enzyme is α-amylase.

As explained herein before, the present invention offers the advantage that the impact of enzymes and oxidoreductants on the dough is delayed until the start of the proving process. Thus, these functional ingredients exert their desired effect during or after proving, thereby avoiding or reducing problems with e.g. stickiness, water holding capacity and dough strength. As regards the aforementioned hydrocolloids it is also advantageous to delay the thickening/gelling effect of these hydrocolloids until after the start of the proving process. If the hydrocolloids start to exert their effect during the admixing of the principal dough components, a relatively viscous mass is obtained that is difficult to handle. In case a gel-forming hydrocolloid is used, the actual mixing operation and/or subsequent handling may disrupt the gel-structure, thereby annihilating the desired functionality of such a gelling hydrocolloid.

In a particularly preferred embodiment of the invention the functional bakery ingredient in the present granule is an enzyme. The term enzyme as used in here refers to any preparation of enzyme at any level of purity, so long as the preparation is enzymatically active. The term enzyme also encompasses a preparation exhibiting a plurality of different specific enzymatic activities.

The benefits of the present invention are particularly pronounced if the functional bakery ingredient(s) are released from the granule during the proving step rather than during the preceding mixing or the subsequent baking step. In order to achieve this, it is preferred to employ a triglyceride fat displaying a slip melting point in the range of 30-40°C. More preferably the triglyceride fat has slip melting point in the range of 33-40°C. Most preferably the slip melting point is in the range of 34-38°C. In particular in case the functional bakery ingredient(s) comprise one or more enzymes, it is very advantageous to design the lipophilic layer in such a way that substantially all of the encapsulated enzyme is released during proving as the activity of most enzymes will decline rapidly during the course of the baking process.

In a preferred embodiment, the lipophilic layer, comprising the combination of triglyceride fat and release agent, has a slip melting point in the range of 30-40°C, more preferably in the range of 33-40°C and most preferably in the range of 34-38°C.

As explained above, the granules of the present invention may advantageously be applied in doughs to achieve a controlled release of one or more functional bakery ingredient(s). The granules of the invention combine the capacity to delay the release of the one or more functional ingredients with the ability to release these ingredients within a relatively short time interval. Since the duration of the proving can be rather short (e.g. about 15-20 minutes) the swift release of functional ingredients that are meant to exert their effect during proving is very advantageous.

In order to facilitate the swift release of the one or more bakery ingredients, it was found to be advantageous to additionally incorporated into the core of the granule a hygroscopic component. Usually such a hygroscopic component is incorporated in a weight ratio of hygroscopic component : functional bakery ingredient(s) in the range of 1:2 to 20:1, preferably of 1:1 to 10:1. Examples of hygroscopic components that may suitably be used to accelerate the release of the functional ingredient(s) include xanthan gum, guar gum, locust been gum, carrageenan, alginate, pectin, CMC, HPMC, starches, dextrins, sugar, salts and combinations of these components. It is noted that, although the hygroscopic component may be a functional bakery ingredient, in accordance with the present invention the hygroscopic component is not an enzyme, an oxidoreductant, an acidulant, or yeast. Particularly preferred are hygroscopic components that swell as a result of absorption of water, especially thickening and gelling agents. Since the formation of a gel structure may hinder the effective release of the functional ingredients, the present granule most preferably contains a thickening agent, e.g. guar gum or locust bean gum.

Like the release agent and the optional hygroscopic component, also the triglyceride fat employed in the present granule has an important impact on the release characteristics. The triglyceride fat also has an important impact on the stability of the granule, especially during storage and handling. It is important that the lipophilic layer is strong enough to withstand handling and mixing. In addition the lipophilic layer should not be sticky as otherwise the granules will agglomerate which will hamper dosing of the granules. In order to enable the preparation of granules that are free flowing, that are storage stable and that survive normal mixing operations, it is advantageous to employ a triglyceride fat that displays an N-profile of N₂₀ > 50; 10 ≤ N₃₀ ≤ 60; and N₄₀ < 5. Preferably the triglyceride fat displays an N₃₀ <50, even more preferably an N₃₀ < 40; and an N₄₀ < 2. Furthermore, the triglyceride fat preferably displays an N₃₀ > 20, more preferably an N₃₀ > 30. The N-profile refers to the solid fat content in the triglyceride fat at the indicated temperature (N₄₀ refers to the solid fat content at 40°C) and is determined by means of pulse NMR.

The triglyceride fat in the lipophilic layer may comprise unmodified, hydrogenated, fractionated and/or interesterified triglycerides. Examples of particularly suitable triglyceride fats include palm mid fractions, palm kernel stearine, cocoa butter and butteroil stearine. Preferably the triglyceride fat contains at least 50 wt.%, more preferably at least 80 wt.% of one or more of these fats or interesterified blends of these fats.

In order to achieve highly desirable release characteristics, it is recommendable that the core constitutes between 10 and 99 wt.% of the granule. More preferably the core constitutes between 20 and 95 wt.% of the granule.

The core of the present granule preferably has a diameter of at least 30 µm, more preferably of at least 50 µm. Generally the diameter of the core will not exceed 800 µm, more preferably it will not exceed 500 µm and most preferably it will not exceed 200 µm. The lipophilic layer will usually have a thickness of at least 2 µm, preferably at least 5 µm, most preferably of at least 10 µm. Normally the thickness of the lipophylic layer will not exceed 200 µm. Preferably the thickness of said layer does not exceed 100 µm, more preferably it does not exceed 70 µm.

In order to further stabilise the present granule and also to improve its free flowing characteristics, it can be advantageous to apply an additional exterior coating containing at least 50 wt.% of an agent selected from the group consisting of sugar, dextrin, tri-calciumphosphate, silicate, calcium carbonate and combinations thereof. More preferably said coating contains at least 70 wt.%, most preferably it contains at least 80 wt.% of such an agent.

The granule of the present invention suitably has a diameter in the range of 10-1000 µm, preferably of 30-500 µm and more preferably of 60-400 µm. Most preferably the diameter of the present granule is within the range of 80-300 µm.

Another aspect of the invention relates to a composition comprising granules as described above, wherein the average diameter of the granules is in the range of 10-1000 µm, more preferably within the range of 30-500 µm and most preferably within the range of 60-300 µm. In order to achieve desirable release characteristics it is advantageous that the particle size distribution of the granules is relatively narrow. Typically, at least 90% of the particles has a particle size within the range of 20-300 µm, more preferably within the range of 30-200 µm and most preferably within the range of 40-100 µm.

As mentioned herein before the present granules and granule containing compositions of the invention exhibit the highly advantageous property that they quickly release the functional bakery ingredient(s) contained therein above a certain temperature in the presence of water. Typically, at least 50 wt.% of the functional bakery ingredient contained in the granule is released within 10 minutes when said granule is immersed in water of a temperature of 38°C. In order to test whether a granule containing composition meets this criterion, a sample equivalent to 75 mg core material should be introduced in a test tube containing 15 ml buffer (0.05M sodium acetate, pH 5.2) at 7 °C. The tube is gently rotated head over tail for 10 minutes at 7 °C. Subsequently, the tube is immersed in a water batch of 38 °C for 10 minutes. After the 10 minutes have passed, the tube is quickly cooled in ice-water. Next, the contents of the tube are immediately passed through a funnel filled with glass wool to remove any remaining granules. The released amount of the functional bakery ingredient is calculated by dividing the amount recovered in the filtered liquid by the total amount present in the original sample.

The present composition may essentially consist of granules as defined herein before, or alternatively, it may contain a combination of said granules and other bakery ingredients. Preferably these ingredients are also in a particulate form so that the total composition displays free flowing behaviour. Examples of additional bakery ingredients that may be incorporated in the present composition include redox agents, emulsifiers, hydrocolloids, flour, salts, malt flour, malt extract, gluten and starch.

Yet another aspect of the invention relates to the use of the aforementioned composition in the preparation of a dough, preferably in the preparation of a bread dough. The dough may simply be prepared by mixing the present composition with the other dough components, e.g. flour, water and yeast. Usually the present composition is incorporated in an amount of between 0.01 and 5% by weight of the dough.

The invention also encompasses a method for the preparation of granules as described above. The present granules may be prepared by a variety of processes including, for example, fluidised bed coating and spray chilling, fluidised bed coating being most preferred. In a preferred embodiment, the present method comprises the steps of:
a. preparing a plurality of particles with a diameter of at least 5 µm, said particles containing one or more functional bakery ingredients selected from the group of enzymes, oxidoreductants, acidulants, hydrocolloids, starches, yeast, sugars, water and flavours;
b. preparing a blend containing at least 50 wt.% of a triglyceride fat with a slip melting point of at least 30°C and at least 1 wt.% of a release agent selected from the group of monoglycerides, diglycerides, datems, lactems, citrems, stearyl-lactylates, polyglycerol esters, lecithins, sucrose esters, fatty acids, soaps and combination thereof; and
c. spraying the blend obtained from step b. in melted form onto the plurality of particles obtained from step a. to achieve encapsulation of the particles with a substantially continuous layer of the said blend; and
d. cooling the resulting encapsulated particles to obtain a plurality of encapsulated particles that exhibit free flowing behaviour.

In step c., when the blend is sprayed onto the plurality of particles, said particles preferably have a temperature that is significantly lower than the slip melting point of the blend. Thus, the blend will start to solidify onto said particles, making it easier to maintain the fluidised bed conditions during the coating process. Preferably the temperature of the particles is at least 2 °C, more preferably at least 5 °C below the slip melting point of the blend. At the same time, the temperature of the particles preferably is not more than 20 °C, more preferably not more than 15 °C below the slip melting point of the blend.

In a particularly preferred embodiment the plurality of particles is prepared in step a. by spray drying, preferably by spray drying the functional bakery ingredient(s) together with a hygroscopic component as defined herein before.

The invention is further illustrated by means of the following examples.

### EXAMPLES

### Example 1

Fungamyl® 1600 bakery granulate (a commercial α-amylase preparation obtained from Aspergillus oryzae; Novo Nordisk) is coated on a fluidised bed laboratory unit (GPCG 1.1, Glatt) with Wurster geometry.

Fungamyl 1600 is fluidised by air. A fat blend consisting of 90 wt.% of a hydrogenated stearin fraction of palm kernel oil (slip melting point of 35°C) and 10 wt.% soy lecithin is molten and sprayed onto the fluidised granulate. Airflow, bed temperature, fat temperature and flow rate, atomisation air pressure and temperature are controlled in such a way that a closed fat film around the granulate particle is formed. The bed temperature is maintained at a sufficiently high temperature to prevent that the fat solidifies before wetting the granulate, leading to free fat particles and uncoated granulate, and a sufficiently low temperature to prevent the bed from agglomerating as a results of the formation of sticky particles.

Two experiments are carried out in which the granulate is coated with different amounts of the fat blend. The amount of fat in the final product is determined by means of low resolution NMR. In both cases the value so obtained is in close agreement with the one calculated from the total amount of fat that was sprayed onto the granulate. The two encapsulates obtained are found to contain approximately 50 wt.% and 75 wt.% of the fat blend.

The particle size distribution is measured via static light scattering. The uncoated enzyme granulate exhibits an average diameter of about 150 µm. The coated granules containing about 50 wt.% fat blend display an average diameter of about 310 µm, whereas the coated granules containing about 75 wt.% of the same blend display an average diameter of about 490 µm.

The stability of the coated granules in an aqueous environment is assessed by suspending the granules in demineralised water of 24°C and measuring the electric conductivity as a function of time. The curves obtained show a rapid increase in conductivity that flattens off within 2-10 minutes, indicating that in both cases a minor amount of the granulate has not been encapsulated perfectly. The granules containing 50 wt.% of fat blend show a significantly faster initial release than the granules containing 75 wt.% of fat blend. The plateau in the curves is achieved at a significantly lower conductivity for the 75 wt.% product than the 50 wt.% product, indicating that the encapsulation with 75 wt.% fat blend is more effective.

The coated granules are again suspended in demineralised water of 24°C and the electric conductivity is measured while the temperature of the water is increased at a rate of about 3 °C/minute. In both cases a sharp increase in conductivity is observed at a temperature close to the melting point of the fat.

The temperature dependency of the release characteristics of the coated granules containing 75 wt.% fat blend is determined by suspending 300 mg of these granules in 4 different tubes containing 15 ml of an aqueous buffer (0.05M sodium acetate buffer, pH 5.2) at 7°C; and 75 mg of the original uncoated granulate in another tube containing 15 ml of the same buffer (control). The tubes are gently rotated head over tail at 7°C for 10 min. Then, the tubes are immersed in different water-baths of 25, 30, 35 and 45°C respectively. After 15 minutes in the water bath, each suspension is quickly cooled in ice-water. Subsequently, the suspensions are centrifuged and filtered and the enzyme activity in the filtered solution is measured. Results show that the suspension that was kept in a water bath at 45°C exhibits the same enzyme activity as the control sample (kept under the same conditions), meaning that effectively all of the encapsulated enzyme was released. Furthermore, it is found that, in the suspension that was kept at 35°C, a major fraction of the enzyme activity has been released. The other 2 suspensions, i.e. those that were kept at 30 and 25°C, only release a minor fraction of the enzyme activity during equilibration at these elevated temperatures.

### Example 2

Example 1 is repeated using a fat blend consisting of 90 wt.% of a hydrogenated .. fraction of palm kernel oil (slip melting point of 35°C) and 10 wt.% of a distilled monoglyceride (Monomuls 90 ex Cognis) with a melting point of about 40 °C. The coated granulates obtained display comparable properties to the coated granulates described in example 1.

## Claims

1. A granule suitable for use in the preparation of a dough, comprising:
a. a hydrophilic core with a diameter of at least 5 µm, said core containing one or more functional bakery ingredients selected from the group of enzymes, oxidoreductants, acidulants, hydrocolloids, starches, yeast, sugars, water and flavours; and
b. a lipophilic substantially continuous layer encapsulating the core, which layer contains at least 50 wt.% triglyceride fat with a slip melting point of at least 30°C and at least 1 wt.% of a release agent selected from the group of monoglycerides, diglycerides, datems, lactems, citrems, stearyl-lactylates, polyglycerol esters, lecithins, sucrose esters, fatty acids, soaps and combination thereof.

2. The granule according to claim 1, wherein the functional bakery ingredient is an enzyme.

3. The granule according to claim 2, wherein the core contains an enzyme selected from the group consisting of α-amylase, β-amylase, xylanase, hemi-cellulase, cellulase, lipase, protease, glucose oxidase, redoxidase, lipoxygenase, peroxidase, ferrulic acid esterase, pullulanase, invertase, mananase, galactomananase, lactase and combinations thereof.

4. The granule according to any one of the preceding claims, wherein the triglyceride fat displays a slip melting point in the range of 30-40°C.

5. The granule according to any one of the preceding claims, wherein the core additionally contains a hygroscopic component, the weight ratio of hygroscopic component : functional bakery ingredient(s) being in the range of 1:2 to 20:1.

6. The granule according to claim 3 or 4, wherein the hygroscopic component is selected from the group consisting of xanthan gum, guar gum, locust been gum, carrageenan, alginate, pectin, CMC, HPMC, starches, sugar, salts and combinations thereof.

7. The granule according to any one of the preceding claims, wherein the release agent has an HLB in the range of 0-12, more preferably in the range of 1-10.

8. The granule according to any one of the preceding claims, wherein the triglyceride fat displays an N-profile of N₂₀ > 50; 10 ≤N₃₀ ≤60; and N₄₀ < 5.

9. The granule according to any one of claims 1-8, wherein the lipophilic layer displays a Stevens hardness of at least ..... at 25°C.

10. The granule according to any one of the preceding claims, wherein the granule comprises an exterior coating containing at least 50 wt.% sugar.

11. The granule according to any one of the preceding claims, said granule having a diameter in the range of 10-1000 µm, preferably of 30-500 µm.

12. A composition comprising granules according to any one of the preceding claims, wherein the average diameter of the granules is in the range of 30-500 µm, preferably in the range of 60-400 µm.

13. The composition according to claim 12, wherein the composition further comprises one or more bakery ingredients selected from the group consisting of redox agents, emulsifiers, hydrocolloids, flour, salts, malt flour, malt extract, gluten and starch.

14. Use of the composition according to claim 12 or 13 in the preparation of a dough, preferably a bread dough.

15. A dough comprising between 0.01 and 5 wt.% of a composition according to claim 12 or 13.

16. A method of manufacturing a composition according to claim 12 or 13, said method comprising the steps of:
a. preparing a plurality of particles with a diameter of at least 5 µm, said particles containing one or more functional bakery ingredients selected from the group of enzymes, oxidoreductants, acidulants, hydrocolloids, starches, yeast, sugars, water and flavours;
b. preparing a blend containing at least 50 wt.% of a triglyceride fat with a slip melting point of at least 30°C and at least 1 wt.% of a release agent selected from the group of monoglycerides, diglycerides, datems, lactems, citrems, stearyl-lactylates, polyglycerol esters, lecithins, sucrose esters, fatty acids, soaps and combination thereof; and
c. spraying the blend obtained from step b. in melted form onto the plurality of particles obtained from step a. to achieve encapsulation of the particles with a substantially continuous layer of the said blend; and
d. cooling the resulting encapsulated particles to obtain a plurality of encapsulated particles that exhibit free flowing behaviour.
